# FASCICULE DE BREVET EUROPEEN

(11) **EP 2 167 220 B1**
(45) Date de publication et mention de la délivrance du brevet: **23.03.2011**
(21) Numéro de dépôt: 08806069.4
(22) Date de dépôt: 24.06.2008
(51) Int. Cl.: B01D 53/32

(54) **TRAITEMENT DE GAZ PAR PLASMA DE SURFACE**
OBERFLÄCHENPLASMAGASVERARBEITUNG
SURFACE PLASMA GAS PROCESSING

(30) Priorité: 06.07.2007 FR 0704900
(43) Date de publication de la demande: 31.03.2010
(73) Titulaire: Ecole Polytechnique, 91128 Palaiseau Cedex (FR); Centre National de la Recherche Scientifique, 75794 Paris Cedex 16 (FR)
(72) Inventeur: ROUSSEAU, Antoine, F-75002 Paris (FR); ALLEGRAUD, Katia, F-95880 Enghien Les Bains (FR); GUAITELLA, Olivier, F-91440 Bures Sur Yvette (FR)
(74) Mandataire: Cabinet Plasseraud
(86) Numéro de dépôt international: PCT/FR2008/051137
(87) Numéro de publication internationale: WO 2009/007588

(56) Documents cités:
- WO-A-2007/051912
- FR-A- 2 904 656
- JP-A- 3 275 119
- JP-A- 11 347 342
- KR-A- 20040 043 274
- US-A1- 2005 118 079
- US-A1- 2006 280 667

## Description

La présente invention concerne une unité de traitement de gaz par plasma de surface, un dispositif qui comprend au moins une telle unité ainsi qu'un procédé de fabrication de l'unité de traitement.

Des normes antipollution sont mises en oeuvre, notamment au niveau européen, qui sont toujours plus contraignantes. Ces normes sont imposées aux industriels, qui doivent les intégrer dans la conception de nouvelles unités de production. Elles répondent aussi à une préoccupation croissante de la population pour des enjeux environnementaux, en même temps que le niveau de vie augmente régulièrement. Toutefois, les procédés de dépollution qui sont utilisés ou qui sont destinés à l'être doivent avoir une consommation énergétique la plus basse possible, notamment lorsque la concentration de polluants dans les effluents à traiter est faible ou très faible. Dans de tels cas, les procédés de dépollution doivent être d'autant plus efficaces que les polluants sont plus dilués.

Les procédés de dépollution qui sont déjà utilisés peuvent être classés en deux catégories, selon qu'ils mettent en oeuvre ou non un plasma.

Les procédés qui mettent en oeuvre un craquage thermique des polluants, une réaction catalytique, une adsorption ou une cryo-condensation des polluants, ou encore ceux qui utilisent des biofiltres, ne mettent pas en oeuvre de plasma. Ils utilisent essentiellement des techniques d'oxydation des polluants, ou bien des techniques de piégeage de ceux-ci. Les techniques d'oxydation, qu'elles soient purement thermiques (craquage thermique) et/ou réalisées en présence d'un catalyseur (oxydation catalytique), nécessitent de chauffer le gaz à plusieurs centaines de degrés. Elles sont surtout efficaces pour des concentrations de polluants qui sont importantes. Mais leur coût énergétique est très élevé, et il devient prohibitif lorsqu'il s'agit de traiter des polluants présents en faible concentration. Or, il peut être nécessaire d'éliminer des polluants même très dilués, notamment lorsque ceux-ci sont particulièrement toxiques ou dégradants pour l'environnement. Les techniques de piégeage (ou d'adsorption) nécessitent en général une maintenance coûteuse. En outre, elles ne réalisent pas une élimination des polluants, mais permettent seulement de les extraire en les fixant sur un support. Lorsque le support est saturé, il doit être changé. La manipulation du support est alors délicate, à cause de la quantité importante de polluants qu'il contient alors. Puis la question du retraitement du support apparaît, ou bien celle d'un stockage en décharge ou d'une destruction de celui-ci lorsqu'il n'est pas recyclable. Enfin, les biofiltres contiennent des micro-organismes qui sont capables de consommer des composés organiques volatils. Les polluants concernés sont alors effectivement éliminés, mais les biofiltres sont eux-mêmes très encombrants, et nécessitent un entretien délicat pour maintenir les micro-organismes en vie.

Comparativement, les procédés qui mettent en oeuvre un plasma ont des coûts énergétiques et des coûts d'entretien qui sont plus faibles. En particulier, l'utilisation de plasmas à pression atmosphérique est compatible avec un grand nombre d'applications. Notamment, les plasmas dits froids permettent une destruction économique de nombreuses espèces polluantes. Mais ils aboutissent souvent à la production de composés toxiques tels que, notamment, des oxydes d'azote (NOₓ), du monoxyde de carbone (CO) ou d'autres composés organiques volatils.

Pour palier ces inconvénients, il a été proposé de combiner l'utilisation d'un plasma avec celle d'un catalyseur qui permet de mieux contrôler les réactions chimiques qui sont mises en oeuvre, et donc de sélectionner des produits de décomposition qui ne sont pas eux-mêmes toxiques ou polluants. Les dispositifs appelés «packed bed reactor» ont été développés en premier. Ils possèdent un réacteur cylindrique qui est rempli de billes d'un matériau catalyseur. Mais ils sont volumineux, et opposent une perte de charge élevée contre la circulation dans le réacteur d'un flux gazeux à traiter. En outre, leur configuration les rend peu appropriés pour des assemblages en série ou en parallèle, notamment afin de traiter des flux gazeux plus importants.

Par ailleurs, des dispositifs de décharge électrique à barrières diélectriques ont été développés. Dans de tels dispositifs, l'apparition d'un arc électrique est inhibée grâce à un isolant diélectrique qui est placé entre les électrodes de décharge. Il n'est alors pas nécessaire d'avoir une géométrie des électrodes qui soit dissymétrique pour maintenir un régime permanent de plasma froid, contrairement aux décharges couronnes. En revanche, l'alimentation électrique ne peut pas être continue (DC), car une accumulation de charges électriques apparaît très rapidement sur le diélectrique et annihile un champ électrique stationnaire qui est créé entre les électrodes. Un plasma qui est ainsi alimenté par une tension électrique continue en présence d'une barrière diélectrique s'éteint donc tout de suite. Mais les avantages de ces dispositifs de décharge à barrières diélectriques sont nombreux :
- la barrière diélectrique procure une sécurité supérieure et une répartition plus homogène du plasma ;
- un circuit de fluide refroidissant peut éventuellement être incorporé dans la barrière diélectrique ;
- ce type de réacteur peut être agrandi facilement, notamment pour obtenir des capacités de traitement qui sont adaptées pour des applications industrielles ; et
- ils sont compatibles avec un fonctionnement à pression atmosphérique.

Pour ces raisons, les dispositifs de décharge à barrières diélectriques sont très utilisés. La production d'ozone, des traitements de surfaces, la production de rayonnement ultraviolet dans les lampes à excimères et la production de rayonnement infrarouge dans les lasers CO₂ figurent parmi ces utilisations. De très nombreuses géométries ont été développées pour ces dispositifs, en fonction des particularités de chaque utilisation. En particulier, la configuration de surface qui est appelée «One Atmosphere Uniform Glow Discharge Plasma» (OAUGDP) a été mise au point pour entraîner un écoulement gazeux. Celle qui est appelée «décharge rampante» a été développée initialement pour des applications laser, mais elle est aussi utilisée pour créer des plasmas stables qui s'étendent sur de grandes surfaces, sans apparition d'arcs électriques.

Enfin, de nombreux dispositifs ont aussi été développés plus récemment, qui combinent l'utilisation d'une décharge avec barrière diélectrique et l'utilisation d'un catalyseur, notamment d'un photocatalyseur. Un exemple de dispositif de ce type qui a notamment été étudié pour décomposer du toluène, comprend un fil d'électrode qui traverse un cylindre rempli de billes de verre, elles-mêmes recouvertes d'oxyde de titane (TiO₂) de forme anatase.

Le document US 2005/0118079 décrit un autre dispositif de ce type, qui est adapté pour générer une décharge de surface et qui possède une configuration plane des électrodes. Des billes d'un matériau photocatalyseur sont placées au dessus de l'une des électrodes qui est exposée au gaz à traiter. Mais, dans cette configuration des électrodes et du matériau photocatalyseur, le plasma qui est créé par la décharge de surface n'est que partiellement en contact avec le matériau photocatalyseur. Pour cette raison, le dispositif ne possède qu'une efficacité limitée pour traiter des flux gazeux.

Un but de la présente invention est de proposer un dispositif de traitement d'un gaz, par exemple pour supprimer des polluants présents dans ce gaz, qui ne présente pas les inconvénients des systèmes antérieurs rappelés ci-dessus.

Plus particulièrement, l'invention a pour but de proposer un dispositif de traitement de gaz à faible coût de fabrication, dont la consommation d'énergie en fonctionnement est faible, qui présente une efficacité de traitement élevée, et qui est compatible avec des volumes importants de gaz à traiter.

Pour cela, l'invention propose une unité de traitement d'un gaz, qui comprend :
- un support diélectrique qui possède une face active et une face arrière parallèles ;
- une première électrode qui est portée par la face active du support ;
- une deuxième électrode qui est portée par la face arrière du support, et qui est décalée par rapport à la première électrode selon une direction parallèle au support ; et
- au moins une portion d'un photocalalyseur qui est disposée au dessus de la face active du support, et qui est apte à activer le traitement du gaz lorsque ledit photocatalyseur reçoit un rayonnement.

L'unité est adaptée pour former un plasma de surface au dessus de la face active du support, dans une zone qui s'étend à partir de la première électrode en direction de la deuxième électrode lorsque ces électrodes sont reliées à deux bornes d'une source d'alimentation électrique. Le plasma produit alors le rayonnement qui est reçu par le photocatalyseur.

Selon l'invention, la portion de photocalalyseur est une couche mince qui est située sur la face active du support, et la première électrode est disposée par-dessus une partie au moins de la couche mince de photocatalyseur, d'un côté de cette dernière qui est opposé au support.

Dans le cadre de l'invention, on appelle couche mince une configuration d'une portion de matériau selon laquelle la portion possède une épaisseur qui est très inférieure aux autres dimensions de cette portion. La portion est réalisée sur un support en utilisant un procédé de dépôt lors duquel le matériau est apporté sur une surface du support sous forme gazeuse, liquide ou même plasma. En particulier, une telle couche mince peut être formée à partir de molécules, d'atomes, d'agrégats d'atomes, d'un film de liquide, de gouttelettes, etc., qui sont amenés individuellement sur le substrat. L'utilisation d'un tel procédé de dépôt de couche mince pour le matériau photocatalyseur d'une unité de traitement selon l'invention est particulièrement économique, et permet de fabriquer l'unité avec un prix de revient qui est bas.

En outre, une unité de traitement selon l'invention peut avoir des dimensions quelconques, qui peuvent être sélectionnées en fonction du débit du gaz à traiter. En particulier, des unités de grandes dimensions peuvent être réalisées simplement, qui sont adaptées pour des utilisations industrielles. En effet, les différents éléments qui constituent l'unité de traitement peuvent être facilement fabriqués en grandes tailles, y compris la portion de photocatalyseur en couche mince.

Un traitement de gaz qui est réalisé au moyen d'une unité selon l'invention combine l'utilisation d'un plasma froid de surface à pression atmosphérique et celle d'un photocatalyseur. Le plasma de surface constitue une source stable du rayonnement qui active le catalyseur. Ce dernier active alors à son tour une transformation de polluants qui sont amenés à son contact. Le traitement de gaz qui est ainsi réalisé peut être une dépollution au moins partielle du gaz, notamment par rapport à des composés organiques volatils qui sont initialement présents dans ce gaz, une réduction d'une odeur du gaz, un traitement bactéricide, ou une combinaison de deux au moins desdits traitements. En outre, étant donné que l'unité de traitement est légère et présente un encombrement faible, elle peut être facilement installée dans un atelier, dans un domicile, dans un véhicule, un avion ou un sous-marin, notamment en étant intégrée dans un système de ventilation ou de climatisation. Enfin, elle est adaptée pour réaliser un traitement gazeux aussi bien en milieu ouvert qu'en milieu fermé ou confiné.

L'unité de traitement de l'invention possède une efficacité de traitement qui est particulièrement élevée. En effet, grâce à la configuration du photocalyseur en couche mince portée par le substrat, ce dernier portant aussi les électrodes, le photocalyseur possède une surface de contact avec le plasma de décharge électrique qui est importante. Simultanément, le photocatalyseur possède un quotient de sa surface par rapport à son volume qui est très élevé. De cette façon, l'interaction du plasma avec le photocatalyseur est très développée. Cette interaction est encore plus favorisée par la disposition de la première électrode par dessus la couche du photocatalyseur. Pour cela, un bord de la première électrode qui est orienté vers la zone du plasma peut être disposé par-dessus la couche mince de photocatalyseur. Ainsi, le plasma de surface est directement généré au niveau de la surface de la couche de photocalyseur, à partir du bord de l'électrode.

En outre, une unité de traitement de l'invention peut être réalisée de façon particulièrement simple lorsque la couche mince de photocatalyseur est continue entre la première électrode et le support. En effet, dans ce cas, la première électrode est formée par dessus la couche mince de photocatalyseur, sans retrait préalable de parties de cette dernière.

Le photocalyseur qui est ainsi mis en oeuvre permet en particulier de décomposer efficacement des composés organiques volatils, sans produire de molécules de rejet qui soient elles-mêmes toxiques ou polluantes, ou en en produisant dans des proportions qui sont suffisamment faibles pour être sans effet néfaste.

Un autre avantage particulièrement remarquable de l'invention est que, étant donné que la couche mince de photocalyseur est disposée au moins en partie entre la première électrode et le substrat, elle provoque un abaissement de la tension d'amorçage du plasma de décharge de surface. En effet, la couche de photocatalyseur possède une permittivité diélectrique qui est en général plus élevée que celle du support, si bien qu'elle participe à renforcer le champ électrique effectif qui intervient au moment de l'allumage du plasma. Etant donné que la tension électrique d'allumage d'un plasma est en général supérieure à la tension électrique qui est nécessaire ultérieurement pour entretenir ce plasma, cette réduction de la tension d'allumage permet de simplifier considérablement la source d'alimentation électrique qui est utilisée avec l'unité de traitement. Le coût global d'installation et d'utilisation d'un dispositif de traitement de gaz qui incorpore une unité selon l'invention est réduit en conséquence. En outre, le bruit électromagnétique qui est généré par le dispositif est aussi réduit.

Différents perfectionnements de l'invention peuvent être introduits dans divers modes de réalisation, séparément ou en combinant certains d'entre eux. Parmi ces perfectionnements, on peut citer les deux suivants :
- l'unité de traitement peut être adaptée pour former en outre un autre plasma de surface au niveau de la face arrière du support, au moins une autre portion d'un photocalalyseur étant disposée sur cette face arrière et apte à activer un traitement d'un gaz lorsque cette autre portion de photocatalyseur reçoit un rayonnement qui est produit par l'autre plasma ; et
- une troisième électrode peut être portée par la face active du support, qui est décalée par rapport à la deuxième électrode selon la direction parallèle au support dans un sens opposé à la première électrode, et qui est adaptée pour augmenter la zone du plasma lorsqu'une tension électrique est appliquée en outre entre les deuxième et troisième électrodes.

L'invention propose en outre un dispositif de traitement d'un gaz qui comprend :
- au moins une unité de traitement telle que décrite précédemment ;
- des moyens de guidage d'un écoulement du gaz qui sont adaptés pour guider le gaz contre la couche mince de photocalalyseur dans la zone du plasma ; et
- une source d'alimentation électrique qui est reliée aux première et deuxième électrodes.

Un tel dispositif, qui est destiné à fonctionner avec un gaz à traiter, est particulièrement léger et facile à installer. En particulier, étant donné que le gaz est traité directement à pression atmosphérique, aucune pompe ni aucune conduite étanche à basse pression n'est nécessaire.

De préférence, la source d'alimentation électrique est adaptée pour produire un signal qui est variable cycliquement entre deux polarités opposées. De cette façon, des accumulations de charges électrostatiques qui sont susceptibles d'apparaître sur certaines parties de l'unité de traitement, notamment sur le support diélectrique et/ou sur la portion de photocatalyseur, sont réduites ou neutralisées. Un fonctionnement permanent de l'unité de traitement est alors facilité.

Si nécessaire, en fonction des dimensions de chaque unité de traitement par rapport au débit du gaz à traiter, plusieurs unités de traitement sensiblement identiques peuvent être disposées côte-à-côte en parallèle dans le dispositif, de sorte que deux unités voisines sont séparées d'une distance qui est adaptée pour que ces deux unités forment ensemble une partie des moyen de guidage de l'écoulement du gaz.

L'invention propose enfin un procédé de fabrication d'une unité de traitement de gaz, qui comprend les étapes suivantes :
/1/ obtenir un film de support à deux faces parallèles ;
/2/ déposer une couche d'un photocalalyseur sur l'une au moins des faces du film de support en utilisant un outil de dépôt de couche mince de ce photocatalyseur ; et
/3/ disposer au moins une première et une seconde portions conductrices électriquement sur l'une et l'autre des faces du film de support, respectivement, ces portions conductrices étant décalées selon une direction parallèle au film, et l'une des deux portions étant disposée au moins en partie par dessus la couche mince de photocalalyseur.

Un tel procédé peut être utilisé pour fabriquer une unité de traitement de gaz telle que décrite précédemment. Les portions conductrices qui sont disposées à l'étape /3/ forment les première et seconde électrodes introduites ci-dessus.

Pour obtenir un prix de revient de l'unité de traitement qui soit particulièrement réduit, le film de support peut posséder initialement une longueur qui correspond à plusieurs unités de traitement. L'étape /2/ est alors réalisée de façon continue lors d'un défilement du film de support dans l'outil de dépôt de la couche mince de photocatalyseur. Puis le film de support est découpé à des dimensions qui correspondent individuellement à des unités de traitement distinctes. Un tel procédé continu présente un rendement et un débit de fabrication particulièrement élevés, notamment parce qu'il évite de recommencer un réglage de l'outil de dépôt de couche mince à chaque réalisation d'une portion de ce film qui est destinée à une nouvelle unité de traitement de gaz.

D'autres particularités et avantages de la présente invention apparaîtront dans la description ci-après d'exemples de réalisation non limitatifs, en référence aux dessins annexés, dans lesquels :
- les figures 1a et 1b sont des vues respectivement en coupe et en plan d'une unité de traitement de gaz selon un premier mode de réalisation de l'invention ;
- les figures 2a et 2b correspondent respectivement aux figures 1a et 1b pour un second mode de réalisation de l'invention ;
- la figure 3 est un schéma de principe en perspective d'un dispositif de traitement de gaz qui incorpore plusieurs unités de traitement selon l'invention.

Il est entendu que, pour raison de clarté, les éléments qui sont représentés sur ces figures ne sont pas en proportion avec des dimensions réelles ni des rapports de dimensions réelles. Pour ces dimensions, on se reportera aux indications qui sont données dans la suite. En outre, des références identiques qui sont indiquées sur des figures différentes désignent des éléments identiques ou qui ont des fonctions identiques.

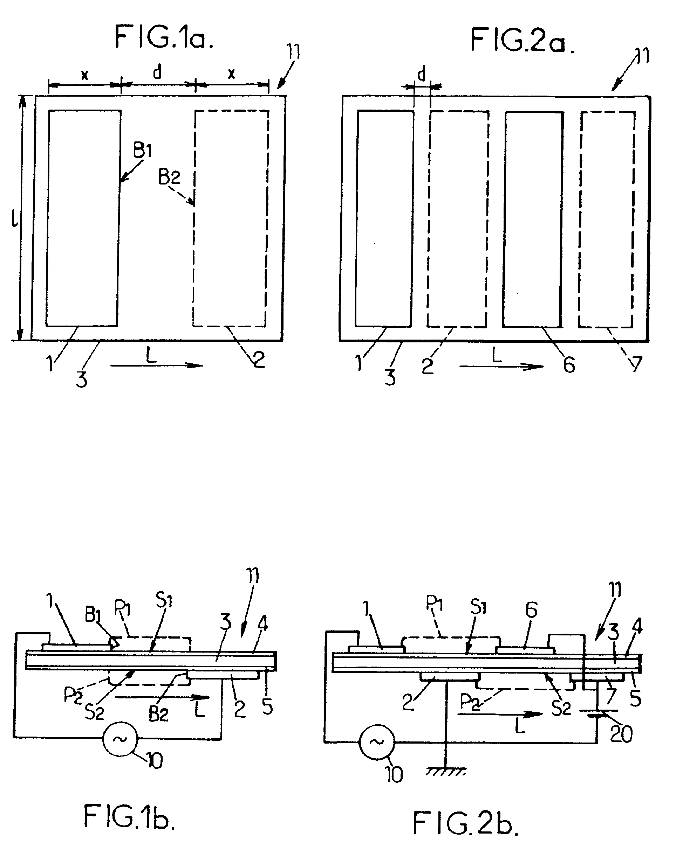

Conformément aux figures 1a et 1b, une unité de traitement de gaz qui est désignée globalement par la référence 11, comprend un support 3 en matériau diélectrique. Ce support 3 est de préférence plan, par exemple carré avec des dimensions de côtés qui peuvent être de 60 cm x 60 cm, par exemple. Il possède une épaisseur réduite, qui peut être de quelques dixièmes de millimètre à quelques millimètres, notamment. Eventuellement, le support 3 peut posséder une structure stratifiée. Il peut comprendre une feuille de verre qui a une épaisseur comprise entre 0,4 et 2,0 mm (millimètre). Alternativement, il peut comprendre un film d'un matériau organique, qui peut être à base de polycarbonate, polyéthylène téréphtalate ou polyimide, par exemple. Dans ce cas, le film organique a été préférablement passivé pour éviter qu'il soit lui-même dégradé lors d'un fonctionnement de l'unité 11.

Les deux faces du support 3 sont notées S1 et S2. Au moins l'une de celles-ci est recouverte d'une couche mince d'un photocatalyseur. Dans l'exemple de mise en oeuvre de l'invention qui est décrit ici, chacune des faces S1 et S2 est recouverte d'une couche mince d'oxyde de titane (TiO₂) référencée 4, resp. 5. D'autres matériaux photocatalyseurs peuvent être utilisés pour les couches 4 et 5, tels que l'oxyde de zinc (ZnO), l'oxyde de nickel (NiO), l'oxyde de chrome (Cr₂O₃) la zircone (ZrO₂) l'oxyde de cérium (CeO₂), etc. De tels matériaux photocatalyseurs possèdent une activité d'oxydo-réduction, qui est particulièrement adaptée pour réaliser une décomposition de composés polluants par oxydation de ceux-ci. Les couches 4 et 5 peuvent avoir chacune une épaisseur qui est comprise entre 10 nm (nanomètre) et 100 (micromètre), de préférence entre 50 nm et 200 nm.

Eventuellement, le support 3 peut comprendre lui-même un substrat et une couche de base qui est portée par celui-ci sur l'une des deux faces S1 et S2, ou bien sur ces deux faces. Une telle couche de base, qui n'est pas représentée sur les figures, forme au moins la face active S1 du support 3 et porte la couche 4. Une telle couche de base peut améliorer un accrochage de la couche 4 sur le substrat, et/ou adapter une texture de la couche 4 de matériau photocatalyseur, par exemple sa rugosité ou sa porosité, pour augmenter une efficacité de traitement de l'unité 11.

Les couches 4 et 5, ainsi que la couche de base éventuelle, sont déposées sur le substrat ou le support 3 en utilisant un outil approprié de dépôt de couche mince, tel qu'une unité de dépôt par pulvérisation cathodique («sputtering» en anglais), par exemple. Un tel outil de dépôt et son fonctionnement sont connus de l'Homme du métier et ne sont pas repris en détail ici. Lorsque le support 3 se présente sous la forme d'un film souple, l'outil de dépôt de la (des) couche(s) mince(s) de photocatalyseur 4 (et 5) peut être agencé en aval d'un rouleau d'alimentation du film de support 3 qui est débobiné. Une telle unité de dépôt est couramment appelée «roll-coater» et permet d'atteindre des débits de production élevés.

Deux portions conductrices parallèles 1 et 2 sont formées sur les faces S1 et S2, respectivement. Par exemple, les portions 1 et 2 sont en oxyde d'étain conducteur (SnOₓ), en argent (Ag) ou en tout autre matériau conducteur stable, et peuvent posséder des épaisseurs qui sont comprises entre quelques nanomètres et plusieurs dizaines de micromètres. Elles s'étendent sensiblement sur toute une largeur I du support 3 et leur dimension x selon une direction commune L qui est parallèle aux faces S1 et S2 et perpendiculaire à la largeur I du support 3 peut être de 10 cm. L'une au moins de ces portions conductrices 1 et 2 peut être formée en utilisant un outil de dépôt de couche mince d'un matériau conducteur électriquement. Alternativement, elle peut être formée par sérigraphie.

Les portions 1 et 2 sont décalées l'une par rapport à l'autre selon la direction L, de sorte que chacune des portions 1, 2 présente un bord qui est orienté vers l'autre portion. Ces bords sont appelés bords internes des portions 1 et 2 et sont référencés respectivement B1 et B2. Ils sont séparés d'une distance d qui peut être comprise entre quelques millimètres et une dizaines de centimètres, parallèlement aux faces S1 et S2.

Dans ces conditions, lorsque les portions conductrices 1 et 2 sont reliées à deux bornes respectives d'une source d'alimentation électrique alternative 10 (figure 1b), une décharge apparaît sur chaque face S1, S2. Cette décharge créé un plasma de surface qui s'étend à partir du bord interne B1, B2 de chaque portion conductrice 1, 2 en direction de l'autre portion conductrice. Sur la figure 1b, les références P1 et P2 désignent les volumes de ces plasmas de surface. Les portions 1 et 2 font alors fonction d'électrodes d'alimentation de chaque plasma. Pour cette raison, elles sont appelées dans la suite électrodes 1, 2. L'alimentation 10 peut être adaptée pour fournir une tension de sortie peu élevée, typiquement 3 kV (kiloVolt), et pouvant être inférieure à 1 kV pour certaines géométries particulières. Cette tension peut être sinusoïdale ou pulsée, avec une fréquence qui est comprise entre quelques Hertz et quelques centaines de kiloHertz.

Chaque électrode 1, 2 est formée par-dessus la couche de matériau photocatalyseur correspondante 4, 5. De préférence, au moins le bord interne B1, B2 de chaque portion 1, 2 est situé par dessus la couche 4, 5 correspondante. Les parties des couches 4 et 5 qui sont comprises entre les électrodes 1 et 2, en projection, ont alors leurs surfaces découvertes qui est située au sein des volumes de plasma P1 et P2. Pour cette raison, l'activité photocatalytique des couches 4 et 5 est utilisée au maximum.

De préférence, une partie au moins de la couche mince de photocatalyseur 4 qui est située entre le support 3 et l'électrode 1 possède une permittivité diélectrique relative supérieure à 6,0. Cette valeur de permittivité diélectrique relative étant supérieure à celle du support 3, il en résulte une réduction de l'épaisseur électrique apparente qui est présente entre les électrodes 1 et 2 lorsqu'elles sont alimentées électriquement. La tension électrique minimale qui est nécessaire pour allumer initialement les plasmas de surface est alors diminuée. Dans le mode de réalisation présent de l'invention, les deux couches 4 et 5 sont identiques, et contribuent donc de façon identique à la réduction de la tension d'allumage des plasmas de surface.

Eventuellement, une troisième portion conductrice 6 peut être agencée sur la face S1, comme représenté sur les figures 2a et 2b. Celle-ci peut être encore identique aux électrodes 1 et 2, et est décalée par rapport à l'électrode 2, selon la direction L dans le sens opposé à l'électrode 1. L'électrode 6 est destinée à être polarisée électriquement par rapport aux électrodes 1 et 2 en utilisant une source de tension continue 20 de quelques centaines de volts. Cela permet d'augmenter l'extension du plasma de surface qui est formé sur la face S1, parallèlement à la direction L, par rapport à l'extension qui résulterait seulement de la distance d de séparation entre les électrodes 1 et 2. De cette façon, une plus grande surface de la couche de matériau photocatalyseur 4 est située dans le volume de plasma P1, et est donc activée par le rayonnement qui est produit par ce plasma. La décharge électrique qui est obtenue est alors appelée décharge rampante, par opposition à une unité 11 à deux électrodes seulement telle que représentée sur les figures 1 a et 1b.

La quatrième électrode 7 possède, vis-à-vis de l'électrode 2 et du plasma de surface qui est créé sur la face S2, une fonction identique à celle de l'électrode 6 vis-à-vis de l'électrode 1 et de la face S1.

Une unité 11 qui correspond aux figures 2a et 2b peut alternativement être mise en oeuvre en tant que réunion de deux unités qui sont similaires chacune à celle des figures 1a et 1b, et qui sont juxtaposées selon la direction L. Dans ce cas, l'électrode 6 a une fonction qui est identique à celle de l'électrode 1, et est reliée avec celle-ci à la première borne de la source d'alimentation alternative 10. De même, l'électrode 7 a une fonction identique à celle de l'électrode 2, et elle est reliée avec cette dernière à la deuxième borne de la source 10. La source de tension de polarisation 20 est supprimée.

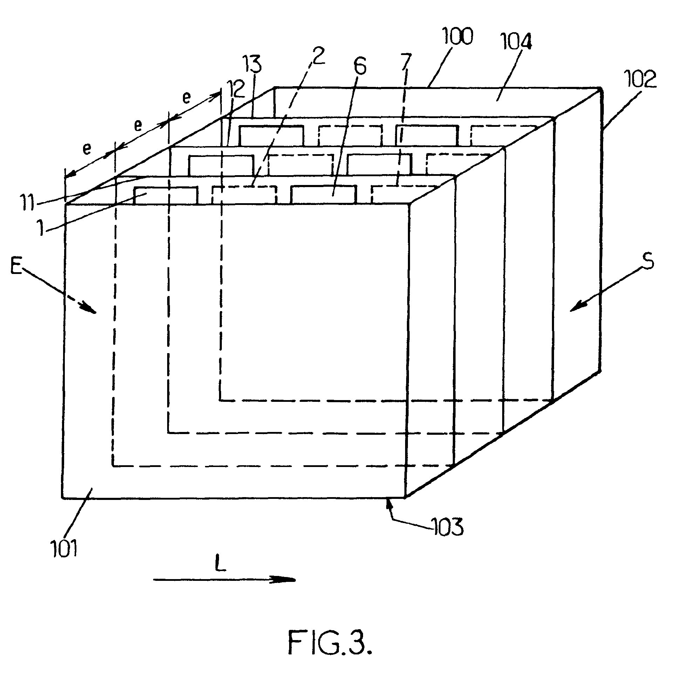

Une telle unité 11 peut être utilisée pour réduire une concentration de polluants qui sont présents dans une atmosphère ambiante. Ces polluants peuvent être des composés organiques volatils, en particulier. Pour cela, plusieurs unités 11-13 peuvent être disposées parallèlement les unes aux autres dans un châssis 100 d'un dispositif de traitement gazeux (figure 3). Ce châssis 100, qui peut être de forme parallélépipédique, est ouvert sur deux faces E et S qui sont opposées, par exemple selon la direction L. Les autres faces 101-104 du châssis 100 sont fermées par des plaques. Un flux gazeux peut alors entrer dans le dispositif par la face E et ressortir par la face S. Entre les deux faces E et S, le flux gazeux passe entre les unités 11-13 qui participent à le guider, en étant séparées par un intervalle e qui peut être compris entre quelques millimètres et quelques centimètres. Eventuellement, plusieurs unités peuvent être disposées dans l'alignement l'une de l'autre, de sorte que le flux gazeux est traité successivement par ces unités lors d'un même écoulement à travers le dispositif.

Les électrodes 1 et 6 d'une part, et 2 et 7 d'autre part de chaque unité 11-13 sont reliées respectivement aux deux bornes de l'alimentation électrique alternative (AC) 10 de la figure 1 a (non reprise sur la figure 3). Des plasmas de surface sont alors créés simultanément sur des portions importantes des deux faces S1 et S2 de toutes les unités 11-13.

Un tel dispositif est adapté pour effectuer un traitement de l'air ambiant, en fonctionnant à pression atmosphérique. Eventuellement, un ventilateur (non représenté) peut être disposé au niveau de l'une des faces E et S, pour provoquer l'écoulement du flux gazeux à travers le châssis 100, entre les unités 11-13. Grâce à la configuration de chaque unité 11-13, un contact important est obtenu entre le gaz qui est destiné à être traité et les plasmas de surface qui sont créés. En particulier et de la façon qui a déjà été expliquée plus haut, la configuration des bords internes de chaque électrode, par dessus la couche de matériau photocatalyseur sous-jacente, favorise une synergie entre le plasma de surface et le photocalyseur. Sur chaque face d'unité 11-13, le plasma s'étend sur toute la longueur des électrodes. Ainsi, le dispositif possède une efficacité de dépollution qui est élevée. En outre, sa consommation énergétique, qui est déterminée par la puissance électrique fournie par l'alimentation 10, est peu élevée.

Enfin, l'utilisation du matériau photocatalyseur des couches 4 et 5 permet de réduire la formation de produits de décomposition qui sont indésirables, en favorisant un processus chimique sélectionné. En particulier, l'utilisation de l'oxyde de titane permet d'augmenter la proportion de dioxyde de carbone (CO₂) dans le gaz traité, pour un grand nombre de composés organiques polluants initialement présents. Une telle décomposition des polluants correspond à une oxydation complète de ceux-ci.

Il est entendu que les modes de réalisation qui viennent d'être décrits en détail peuvent être adaptés de multiples façons, notamment en fonction de l'application considérée, tout en conservant certains au moins des avantages de l'invention. Parmi ces adaptations, on peut citer :
- les électrodes 1 et 2 peuvent être très fines, avec une largeur x de l'ordre de un millimètre, et décalées l'une par rapport à l'autre d'une distance d de quelques millimètres. Chaque support 3 peut alors comporter un grand nombre de paires d'électrodes 1, 2 qui sont disposées successivement le long de l'écoulement du flux gazeux ;
- l'emploi d'un catalyseur approprié permet d'augmenter localement la concentration des polluants au niveau la surface du support. Un traitement du gaz qui est encore plus efficace est obtenu, notamment lorsque les polluants sont présents en très faible concentration ;
- le traitement du gaz peut être réalisé de manière continue ou séquentielle, selon que le gaz est admis sous forme d'un flux continu où que des volumes fixes de gaz sont traités successivement en étant chacun enfermés dans le dispositif de traitement pendant une durée déterminée ;
- le dispositif de traitement de gaz peut comporter en outre une section de décomposition de molécules d'ozone qui sont éventuellement produites pendant le traitement du gaz par les plasmas de surface. Une telle section peut incorporer un matériau poreux tel que l'oxyde de manganèse (MnO₂) ou l'alumine (γ-Al₂O₃) par exemple ; et
- l'écoulement du gaz à traiter peut être orienté de façon quelconque parallèlement au support 3 de chaque unité, par rapport à la direction L de décalage des électrodes 1 et 2.

## Revendications

1. Unité (11) de traitement d'un gaz comprenant :
- un support diélectrique (3) ayant une face active (S1) et une face arrière (S2) parallèle à ladite face active ;
- une première électrode (1) portée par la face active du support ;
- une deuxième électrode (2) portée par la face arrière du support, et décalée par rapport à la première électrode selon une direction parallèle au support (L) ; et
- au moins une portion d'un photocalalyseur (4) disposée au dessus de la face active du support et apte à activer le traitement du gaz lorsque ledit photocatalyseur reçoit un rayonnement,
ladite unité étant adaptée pour former un plasma de surface au dessus de la face active du support (S1), dans une zone (P1) s'étendant à partir de la première électrode (1) en direction de la deuxième électrode (2) lorsque lesdites première et deuxième électrodes sont reliées à deux bornes d'une source d'alimentation électrique (10), et ledit plasma produisant un rayonnement reçu par le photocatalyseur ;
l'unité étant **caractérisée en ce que** la portion de photocalalyseur (4) est une couche mince située sur la face active du support (S1),
et **en ce que** la première électrode (1) est disposée par dessus une partie au moins de la couche mince de photocatalyseur (4), d'un côté de ladite couche mince opposé au support.

2. Unité selon la revendication 1, dans laquelle un bord (B1) de la première électrode (1) orienté vers la zone du plasma (P1) est disposé par-dessus la couche mince de photocatalyseur (4).

3. Unité selon la revendication 1 ou 2, dans laquelle la couche mince de photocatalyseur (4) est continue entre la première électrode (1) et le support (3).

4. Unité selon l'une quelconque des revendications précédentes, dans laquelle la couche mince de photocatalyseur (4) a une épaisseur comprise entre 10 nanomètres et 100 micromètres, de préférence entre 50 nanomètres et 200 nanomètres.

5. Unité selon l'une quelconque des revendications précédentes, adaptée de sorte que le traitement du gaz est sélectionné parmi une dépollution au moins partielle dudit gaz, une réduction d'une odeur dudit gaz, un traitement bactéricide, et une combinaison de deux au moins desdits traitements.

6. Unité selon l'une quelconque des revendications précédentes, adaptée pour former en outre un autre plasma de surface (P2) au niveau de la face arrière du support (S2), au moins une autre portion d'un photocalalyseur (5) étant disposée sur ladite face arrière du support et apte à activer un traitement d'un gaz lorsque ladite autre portion de photocatalyseur reçoit un rayonnement produit par ledit autre plasma.

7. Unité selon l'une quelconque des revendications précédentes, comprenant en outre une troisième électrode (6) portée par la face active du support (S1), décalée par rapport à la deuxième électrode (2) selon la direction parallèle au support (L) dans un sens opposé à la première électrode (1), et adaptée pour augmenter la zone du plasma lorsqu'une tension électrique est appliquée en outre entre les deuxième et troisième électrodes.

8. Unité selon l'une quelconque des revendications précédentes, dans laquelle le support (3) possède une structure stratifiée.

9. Unité selon l'une quelconque des revendications précédentes, dans laquelle le support (3) comprend lui-même un substrat et une couche de base portée par ledit substrat, ladite couche de base formant la face active du support (S1) et portant la couche mince de photocatalyseur (4).

10. Unité selon l'une quelconque des revendications précédentes, dans laquelle le support (3) comprend une feuille de verre ayant une épaisseur comprise entre 0,4 et 2,0 millimètres, ou un film d'un matériau organique.

11. Unité selon l'une quelconque des revendications précédentes, dans laquelle le photocatalyseur (4) est un matériau qui possède une activité d'oxydo-réduction.

12. Unité selon l'une quelconque des revendications précédentes, dans laquelle une partie de la couche mince de photocatalyseur (4) située entre le support (3) et la première électrode (1) possède une permittivité diélectrique relative supérieure à 6,0.

13. Dispositif de traitement d'un gaz comprenant :
- au moins une unité de traitement (11-13) selon l'une quelconque des revendications précédentes ;
- des moyens de guidage d'un écoulement du gaz adaptés pour guider le gaz contre la couche mince de photocalalyseur (4) dans la zone du plasma (P1) ; et
- une source d'alimentation électrique (10) reliée aux première et deuxième électrodes.

14. Dispositif selon la revendication 13, comprenant plusieurs unités de traitement (11-13) disposées côte-à-côte en parallèle, et deux unités voisines dudit dispositif étant séparées d'une distance (e) adaptée pour que lesdites deux unités forment ensemble une partie des moyens de guidage de l'écoulement du gaz.

15. Procédé de fabrication d'une unité de traitement de gaz (11), comprenant les étapes suivantes :
/1/ obtenir un film de support (3) à deux faces parallèles (S1, S2) ;
/2/ déposer une couche d'un photocalalyseur (4) sur l'une au moins des faces du film de support (S1) en utilisant un outil de dépôt de couche mince dudit photocatalyseur ; et
/3/ disposer au moins une première (1) et une seconde (2) portions conductrices électriquement sur l'une et l'autre des faces du film de support (S1, S2), respectivement, lesdites portions conductrices étant
décalées selon une direction parallèle au film (L), et l'une des deux portions étant disposée au moins en partie par dessus la couche mince de photocalalyseur (2).

16. Procédé selon la revendication 15, utilisé pour fabriquer une unité de traitement de gaz (11) selon l'une quelconque des revendications 1 à 12.

17. Procédé selon la revendication 15 ou 16, suivant lequel :
- le film de support (3) possède une longueur correspondant à plusieurs unités de traitement ;
- l'étape /2/ est réalisée de façon continue par défilement du film de support (3) dans l'outil de dépôt de la couche mince de photocatalyseur ; et
- le film de support est ensuite découpé à des dimensions correspondant individuellement à des unités de traitement distinctes.

18. Procédé selon la revendication 17, suivant lequel le film de support (3) est souple, et l'outil de dépôt de la couche mince du photocatalyseur utilisé à l'étape /2/ est agencé en aval d'un rouleau d'alimentation dudit film de support qui est débobiné.

19. Procédé selon l'une quelconque des revendications 15 à 18, suivant lequel l'une au moins des portions conductrices (1, 2) est formée en utilisant un outil de dépôt de couche mince d'un matériau conducteur électriquement, ou par sérigraphie.

## Claims

1. Gas processing unit (11) including:
- a dielectric carrier (3) having an active surface (S1) and a back surface (S2), which is parallel to said active surface;
- a first electrode (1) supported by the active surface of the carrier;
- a second electrode (2) supported by the back surface of the carrier, and which is offset relative to the first electrode along a direction parallel to the carrier (L); and
- at least one portion of a photocatalyst (4) arranged above the active surface of the carrier and capable of activating the gas processing when said photocatalyst receives radiation,
said unit being suitable for forming a surface plasma above the active surface of the carrier (S1), in a region (P1) extending from the first electrode (1) toward the second electrode (2), when said first and second electrodes are connected to two terminals of an electrical power source (10), and said plasma is producing radiation received by the photocatalyst;
the unit being **characterised in that** the photocatalyst portion (4) is a thin layer situated on the active surface of the carrier (S1),
and **in that** the first electrode (1) is arranged over at least a portion of the thin photocatalyst layer (4), on a side of said thin layer opposite the carrier.

2. Unit according to claim 1, wherein an edge (B1) of the first electrode (1), which is oriented towards the plasma region (P1), is arranged over the thin photocatalyst layer (4).

3. Unit according to claim 1 or 2, wherein the thin photocatalyst layer (4) is continuous between the first electrode (1) and the carrier (3).

4. Unit according to anyone of the preceding claims, wherein the thin photocatalyst layer (4) has a thickness of between 10 nanometres and 100 micrometres, preferably between 50 nanometres and 200 nanometres.

5. Unit according to anyone of the preceding claims, adapted so that the gas processing is selected from an at least partial pollutant removal from said gas, an odour reduction of said gas, a bactericidal treatment, and a combination of at least two of said treatments.

6. Unit according to anyone of the preceding claims, suitable for further forming another surface plasma (P2) on the back surface of the carrier (S2), at least one other portion of a photocatalyst (5) being arranged on said back surface of the carrier and capable of activating processing of a gas when said other photocatalyst portion receives radiation produced by said other plasma.

7. Unit according to anyone of the preceding claims, further including a third electrode (6) supported by the active surface of the carrier (S1), which is offset relative to the second electrode (2) along the direction parallel to the carrier (L), in an opposite direction from that of the first electrode (1), and which is suitable for increasing the plasma region when electrical voltage is further applied between the second and third electrodes.

8. Unit according to anyone of the preceding claims, wherein the carrier (3) has a layered structure.

9. Unit according to anyone of the preceding claims, wherein the carrier (3) itself includes a substrate and a base layer supported by said carrier, said base layer forming the active surface of the carrier (S1) and supporting the thin photocatalyst layer (4).

10. Unit according to anyone of the preceding claims, wherein the carrier (3) includes a glass sheet having a thickness of between 0.4 and 2.0 millimetres, or a film made of an organic material.

11. Unit according to anyone of the preceding claims, wherein the photocatalyst (4) is a material which has redox activity.

12. Unit according to anyone of the preceding claims, wherein a portion of the photocatalyst thin layer (4) situated between the carrier (3) and the first electrode (1) has a relative dielectric permittivity greater than 6.0.

13. Gas processing device including:
- at least one processing unit (11-13) according to anyone of the preceding claims;
- gas-flow guiding means suitable for guiding the gas against the thin photocatalyst layer (4) in the plasma region (P1); and
- an electrical power source (10) connected to the first and second electrodes.

14. Device according to claim 13, including several processing units (11-13) arranged side-by-side in parallel, and two adjacent units of said device being separated by a distance (e) adapted so that said two units together form a portion of the gas-flow guiding means.

15. Method for manufacturing a gas processing unit (11), including the following steps:
/1/ obtaining a carrier film (3) having two parallel surfaces (S1, S2);
/2/ depositing a photocatalyst layer (4) on at least one of the surfaces of the carrier film (S1) by using a tool for depositing a thin layer of said photocatalyst; and
/3/ arranging at least one first (1) and one second (2) electrically conductive portions on both surfaces of the carrier film (S1, S2), respectively, said conductive portions being offset in a direction parallel to the film (L), and one of the two portions being arranged at least partially over the thin photocatalyst layer (2).

16. Method according to claim 15, used to manufacture a gas processing unit (11) according to anyone of claims 1 to 12.

17. Method according to claim 15 or 16, according to which:
- the carrier film (3) has a length corresponding to several processing units;
- step /2/ is carried out continuously by having the carrier film (3) running into the tool for depositing the thin photocatalyst layer; and
- the carrier film is then cut-out to dimensions which correspond individually to separate processing units.

18. Method according to claim 17, according to which the carrier film (3) is flexible, and the tool for depositing the thin photocatalyst layer used in step /2/ is arranged downstream from a feed roller for said carrier film which is unwound.

19. Method according to anyone of claims 15 to 18, according to which at least one of the conductive portions (1, 2) is formed either by using a tool for depositing a thin layer of an electrically conductive material, or by means of screen printing.

## Patentansprüche

1. Einheit (11) zur Behandlung eines Gases, aufweisend:
- einen dielektrischen Träger (3), der eine aktive Fläche (S1) und eine zu der aktiven Fläche parallele, hintere Fläche (S2) hat;
- eine von der aktiven Fläche des Trägers getragene erste Elektrode (1);
- eine von der hinteren Fläche des Trägers getragene zweite Elektrode (2), die bezüglich der ersten Elektrode in einer zum Träger parallelen Richtung (L) versetzt angeordnet ist; und
- mindestens einen Abschnitt eines Photokatalysators (4), der oberhalb der aktiven Fläche des Trägers angeordnet und imstande ist, die Behandlung des Gases zu aktivieren, wenn der Photokatalysator eine Strahlung empfängt;
wobei die Einheit angepasst ist, oberhalb der aktiven Fläche (S1) des Trägers in einer Zone (P1), die sich von der ersten Elektrode (1) in Richtung zur zweiten Elektrode (2) erstreckt, ein Oberflächenplasma zu bilden, wenn die erste und zweite Elektrode an zwei Klemmen einer elektrischen Versorgungsquelle (10) angeschlossen sind, und das Plasma eine Strahlung erzeugt, die der Photokatalysator empfängt;
wobei die Einheit **dadurch gekennzeichnet ist, dass** der Photokatalysatorteil (4) eine an der aktiven Fläche (S1) des Trägers angeordnete dünne Schicht ist,
und **dadurch**, dass die erste Elektrode (1) oberhalb mindestens eines Abschnitts der dünnen Photokatalysatorschicht (4) an deren dem Träger gegenüberliegenden Seite angeordnet ist.

2. Einheit nach Anspruch 1, in welcher ein zur Zone des Plasmas (P1) hin gerichteter Rand (B1) der ersten Elektrode (1) oberhalb der dünnen Photokatalysatorschicht (4) angeordnet ist.

3. Einheit nach Anspruch 1 oder 2, in welcher die dünne Photokatalysatorschicht (4) zwischen der ersten Elektrode (1) und dem Träger (3) kontinuierlich ist.

4. Einheit nach einem der vorstehenden Ansprüche, in welcher die dünne Photokatalysatorschicht (4) eine Dicke zwischen 10 Nanometer und 100 Mikrometer, vorzugsweise zwischen 50 Nanometer und 200 Nanometer, hat.

5. Einheit nach einem der vorstehenden Ansprüche, die derart angepasst ist, dass sich die Behandlung des Gases auswählen lässt aus: eine zumindest teilweise Reinigung des Gases, eine Verringerung eines Geruchs des Gases, eine bakterizide Behandlung und eine Kombination aus mindestens zwei der Behandlungen.

6. Einheit nach einem der vorstehenden Ansprüche, die angepasst ist, um außerdem ein anderes Oberflächenplasma (P2) im Bereich der hinteren Fläche (S2) des Trägers zu bilden, wobei mindestens ein anderer Abschnitt eines Photokatalysators (5) an der hinteren Fläche des Trägers angeordnet ist und imstande ist, eine Behandlung eines Gases zu aktivieren, wenn der andere Photokatalysatorteil eine von dem anderen Plasma erzeugte Strahlung empfängt.

7. Einheit nach einem der vorstehenden Ansprüche, aufweisend außerdem eine von der aktiven Fläche (S1) des Trägers getragene dritte Elektrode (6), die bezüglich der zweiten Elektrode (2) in der zum Träger parallelen Richtung (L) in entgegengesetztem Sinn zur ersten Elektrode (1) versetzt angeordnet ist und angepasst ist, um die Zone des Plasmas zu vergrößern, wenn eine elektrische Spannung außerdem zwischen der zweiten und dritten Elektrode angelegt ist.

8. Einheit nach einem der vorstehenden Ansprüche, in welcher der Träger (3) eine Schichtstruktur aufweist.

9. Einheit nach einem der vorstehenden Ansprüche, in welcher der Träger (3) seinerseits ein Substrat und eine von dem Substrat getragene Basisschicht aufweist, wobei die Basisschicht die aktive Fläche (S1) des Trägers bildet und die dünne Photokatalysatorschicht trägt.

10. Einheit nach einem der vorstehenden Ansprüche, in welcher der Träger (3) eine Glasschicht, die eine Dicke zwischen 0,4 und 2,0 Millimeter hat, oder eine Schicht aus einem organischen Material aufweist.

11. Einheit nach einem der vorstehenden Ansprüche, in welcher der Photokatalysator (4) ein Material ist, das eine Oxidation/Reduktion-Aktivität besitzt.

12. Einheit nach einem der vorstehenden Ansprüche, in welcher ein zwischen dem Träger (3) und der ersten Elektrode (1) angeordneter Teil der dünnen Photokatalysatorschicht (4) eine relative Dielektrizitätskonstante größer als 6,0 besitzt.

13. Vorrichtung zur Behandlung eines Gases, aufweisend:
- mindestens eine Behandlungseinheit (11-13) nach einem der vorstehenden Ansprüche;
- geeignete Lenkungseinrichtungen zum Lenken einer Strömung des Gases, um in der Zone des Plasmas (P1) das Gas gegen die dünne Photokatalysatorschicht (4) zu lenken; und
- eine mit den ersten und zweiten Elektroden verbundene elektrische Versorgungsquelle (10).

14. Vorrichtung nach Anspruch 13, aufweisend mehrere parallel nebeneinander angeordnete Behandlungseinheiten (11-13), wobei zwei benachbarte Einheiten der Vorrichtung in einem geeigneten Abstand (e) angeordnet sind, so dass die zwei Einheiten zusammen einen Teil der Lenkungseinrichtungen der Gasströmung bilden.

15. Verfahren zur Herstellung einer Gasbehandlungseinheit (11), aufweisend die folgenden Schritte:
/1/ Bereitstellen einer Trägerfolie (3), die zwei parallele Flächen (S1,S2) aufweist;
/2/ Aufbringen einer Photokatalysatorschicht (4) auf mindestens eine (S1) der Flächen der Trägerfolie unter Verwendung eines Werkzeugs zum Aufbringen einer dünnen Schicht des Photokatalysators; und
/3/ Aufbringen mindestens eines ersten (1) und eines zweiten (2) elektrisch leitfähigen Abschnitts auf die eine bzw. die andere der Flächen (S1,S2) der Trägerfolie, wobei die leitfähigen Abschnitte in einer zur Folie parallelen Richtung (L) versetzt zueinander angeordnet sind und wobei der eine der zwei Abschnitte zumindest teilweise oberhalb der dünnen Photokatalysatorschicht (4) angeordnet ist.

16. Verfahren nach Anspruch 15, das verwendet wird, um eine Gasbehandlungseinheit (11) nach einem der Ansprüche 1 bis 12 herzustellen.

17. Verfahren nach Anspruch 15 oder 16, gemäß welchem:
- die Trägerfolie (3) eine Länge besitzt, die mehreren Behandlungseinheiten entspricht;
- der Schritt /2/ auf kontinuierliche Weise durch Abspulen der Trägerfolie in dem zum Aufbringen der dünnen Photokatalysatorschicht vorgesehenen Werkzeug durchgeführt wird; und
- die Trägerfolie anschließend auf Maße, die einzelnen Behandlungseinheiten entsprechen, zugeschnitten wird.

18. Verfahren nach Anspruch 17, gemäß welchem die Trägerfolie (3) biegsam ist und das in dem Schritt /2/ verwendete, zum Aufbringen der dünnen Photokatalysatorschicht vorgesehene Werkzeug stromabwärts einer Zuführungsrolle der Trägerfolie, die abgespult wird, angeordnet ist.

19. Verfahren nach einem der Ansprüche 15 bis 18, gemäß welchem zumindest der eine der leitfähigen Abschnitte (1,2) unter Verwendung eines zum Aufbringen einer dünnen Schicht eines elektrisch leitenden Materials geeigneten Werkzeugs oder mittels Siebdruck gebildet wird.
